# EUROPÄISCHE PATENTSCHRIFT

(11) **EP 1 289 949 B1**
(45) Veröffentlichungstag und Bekanntmachung des Hinweises auf die Patenterteilung: **05.09.2007**
(21) Anmeldenummer: 01947366.9
(22) Anmeldetag: 13.06.2001
(51) Int. Cl.: C07D 201/08

(54) **VERFAHREN UND HERSTELLUNG VON CAPROLACTAM**
METHOD FOR THE PRODUCTION OF CAPROLACTAM
PROCEDE DE PRODUCTION DE CAPROLACTAME

(30) Priorität: 16.06.2000 DE 10028950
(43) Veröffentlichungstag der Anmeldung: 12.03.2003
(73) Patentinhaber: BASF Aktiengesellschaft, 67056 Ludwigshafen (DE)
(72) Erfinder: OHLBACH, Frank, 40219 Düsseldorf (DE); ANSMANN, Andreas, 69168 Wiesloch (DE); FISCHER, Rolf-Hartmuth, 69121 Heidelberg (DE); MELDER, Johann-Peter, 67459 Böhl-Iggelheim (DE)
(86) Internationale Anmeldenummer: PCT/EP2001/006687
(87) Internationale Veröffentlichungsnummer: WO 2001/096294

(56) Entgegenhaltungen:
- EP-A- 0 023 751
- EP-A- 0 576 976
- GB-A- 2 108 119
- US-A- 4 119 665

## Beschreibung

Die vorliegende Erfindung betrifft ein Verfahren zur Herstellung von Caprolactam aus einer Verbindung der Formel (I)

NC - (CH₂)₄ - CO - R (I)

mit R: Carbonsäureamid-, Carbonsäure- oder Carbonsäureestergruppe
dadurch gekennzeichnet, daß man
a) eine Verbindung (I) oder deren Gemische in Gegenwart von Ammoniak und gegebenenfalls eines flüssigen Verdünnungsmittels (VI) mit Wasserstoff in Gegenwart eines Katalysators (II) wobei als katalytisch aktive Komponente des Ktalysators (II) ein Metall, ausgewählt aus der Gruppe bestehend aus Fe, Ni, Co oder Ru oder deren Gemische, verwendet wird, hydriert unter Erhalt einer Mischung (III),
b) aus Mischung (III) Wasserstoff und Katalysator (II) abtrennt unter Erhalt einer Mischung (IV) und
c) Mischung (IV) gegebenenfalls in Gegenwart eines flüssigen Verdünnungsmittels (VII) in Gegenwart eines Katalysators (V) zu Caprolactam umsetzt.

Verfahren zur Herstellung von Caprolactam aus von Verbindungen der Formel (I) sind an sich bekannt.

So beschreibt DP 915 568 die Umsetzung von Cyanvaleriansäureestern an Raney-Cobalt bei 120°C und 200 bar. Es werden Caprolactam-Ausbeuten von 40-52% erhalten.

In J. Polym. Sci. Part B Polymer Lett. 2 (1964) Nr. 5, Seite 491-3 wird ebenfalls die Umsetzung von Cyanvaleriansäureestern an Raney-Katalysatoren beschrieben. Es werden Caprolactam-Ausbeuten von 31-74% erhalten.

In EP-A-23 751 wird die Hydrierung von Cyanvaleriansäureestern an Ru/Fe-Festbett-Katalysatoren beschrieben. Es werden Caprolactam-Ausbeuten von 35% erhalten.

Gemäß JP 305330 werden Cyanvaleriansäureester zunächst an Raney-Nickel oder Raney-Cobalt hydriert und anschließend ohne Katalysator thermolysiert. Es werden Caprolactam-Ausbeuten von 80-90 % erhalten.

Nachteilig bei diesen Verfahren sind die für technische Erfordernisse zu geringen Ausbeuten oder Raum-Zeit-Ausbeuten.

Der vorliegenden Erfindung lag die Aufgabe zugrunde, ein Verfahren bereitzustellen, das die Herstellung von Caprolactam aus Verbindungen der Formel (I) auf technisch einfache und wirtschaftliche Weise ermöglicht unter Vermeidung der genannten Nachteile. Demgemäß wurde das eingangs definierte Verfahren gefunden.

Erfindungsgemäß werden als Verbindung (I) solche der Formel

NC - (CH₂)₄ - CO - R (I)

mit R: Carbonsäureamid-, Carbonsäure- oder Carbonsäureestergruppe
eingesetzt. Es können auch Gemische solcher Verbindungen eingesetzt werden, die im Sinne der vorliegenden Erfindung ebenfalls als Verbindung (I) bezeichnet werden. Besonders bevorzugt setzte man nur eine Verbindung als Verbindung (I) ein.

Als Carbonsäureamidgruppen kommen vorteilhaft solche der allgemeine Formel -CO-NR¹R² in Betracht, in denen R¹ und R² unabhängig voneinander Wasserstoff oder einen organischen Rest, vorzugsweise C₁-C₄-Alkyl, darstellen. Besonders bevorzugte Carbonsäureamidgruppe ist die Gruppe -CONH₂.

Als Carbonsäureestergruppen kommen vorteilhaft solche der allgemeine Formel -CO-OR³ in Betracht, in denen R³ einen organischen Rest, vorzugsweise C₁-C₄-Alkyl, darstellen. Besonders bevorzugte Carbonsäureestergruppe ist die Gruppe -CO-OCH₃.

Die Herstellung von Verbindungen der Formel (I) ist allgemein bekannt, beispielsweise aus Reppe, Lieb. Ann. Chem. 596 (1955) 127, BE 850 113, EP-A-576976 oder EP-A-1 107 947.

Erfindungsgemäß wird gemäß Schritt a) Verbindung (I) in Gegenwart von Ammoniak und gegebenenfalls eines flüssigen Verdünnungsmittels (VI) mit Wasserstoff in Gegenwart eines Katalysators (II) hydriert unter Erhalt einer Mischung (III).

Vorteilhaft sollten im allgemeinen pro kg Verbindung (I) mindestens 0,1 kg, vorzugsweise 0,1 bis 10, besonders bevorzugt 0,2 bis 5 kg, insbesondere 0,5 bis 5 kg Ammoniak eingesetzt werden.

Schritt a) kann vorteilhaft in Gegenwart eines flüssigen Verdünnungsmittels (VI) durchgeführt werden. Als Verdünnungsmittel (VI) kommen vorzugsweise Wasser oder organische Verdünnungsmittel beispielsweise C₄- bis C₉-Alkanole, wie n-Butanol, i-Butanol, n-Pentanol, Ether, wie Methyl-tert-butyl-ether, Tetrahydrofuran, vorzugsweise aliphatische Kohlenwasserstoffe, wie n-Hexan, cycloaliphatische Kohlenwasserstoffe, wie Cyclopentan oder Cyclohexan, insbesondere aromatische Kohlenwasserstoffe, wie Benzol, Toluol, o-Xylol, m-Xylol, p-Xylol, Ethylbenzol, i-Propylbenzol, Di-i-propylbenzol sowie Gemische solcher Verbindungen, beispielsweise Petrolether, in Betracht. Die Kohlenwasserstoffe können funktionelle Gruppen, wie Halogene, beispielsweise Chlor, tragen, wie in Chlorbenzol.

Als Katalysator (II) kommt vorteilhaft ein heterogener Katalysator in Betracht. Katalysator (II) kann dabei vorzugsweise als Festbettkatalysator eingesetzt werden. Als katalytisch aktive Komponente von Katalysator (II) kommt ein Metall, ausgewählt aus der Gruppe bestehend aus Fe, Ni, Co oder Ru oder deren Gemische in Betracht.

Solche Komponenten können als Vollkatalysator, beispielsweise als Raney-Nickel, Raney-Cobalt oder aus synthetischen oder natürlichen Eisenoxid enthaltenden Materialien, beispielsweise Magnetit, durch Reduktion erhaltenes Eisen, eingesetzt werden.

Solche Komponenten können als Trägerkatalysator eingesetzt werden. Geeignete Trägerkatalysatoren können vorteilhaft durch Tränken oder Besprühen eines Trägermaterials mit einer Lösung, die Verbindungen solcher Metalle oder deren Gemische enthält, oder durch Sprühtrocknung einer Suspension, die Trägermaterial und Verbindungen solcher Metalle oder deren Gemische enthält, erhalten werden. Solche Trägerkatalysatoren können an sich bekannte Trägermaterialien enthalten, vorzugsweise Aluminiumoxid, Siliziumoxid, Alumosilikate, Lanthanoxid, Titandioxid, Zirkondioxid, Magnesiumoxid, Zinkoxid, Zeolithe oder Aktivkohle sowie deren Mischungen.

Vorteilhaft beträgt in Schritt a) der Anteil der Verbindung (I) an der Summe der Ausgangskomponenten Verbindung (I), Ammoniak und Verdünnungsmittel (VI) 0,1 bis 50 Gew.-%, vorzugsweise 1 bis 30 Gew.-%, insbesondere 2 bis 20 Gew.-%.

Die Umsetzung kann vorteilhaft in flüssiger Phase durchgeführt werden.

Die Umsetzung kann vorteilhaft bei Temperaturen von im allgemeinen 40 bis 250°C, vorzugsweise 50 bis 100°C, besonders bevorzugt 60 bis 120°C durchgeführt werden. Der Druck sollte im allgemeinen im Bereich von 1 bis 350 bar, vorzugsweise von 25 bis 250 bar liegen.

Bei der Umsetzung gemäß Schritt a) wird eine Mischung (III) erhalten.

Gemäß Schritt b) wird aus der Mischung (III) Wasserstoff und Katalysator (II) abgetrennt unter Erhalt einer Mischung (IV).

Die Abtrennung des Katalysators (II) kann in an sich bekannter Weise erfolgen.

Im Falle einer Suspensionsfahrweise kann der Katalysator (II) vorteilhaft durch Filtration, insbesondere über eine Membran mit geeigneter Porengröße, erfolgen. Im Falle einer Festbettfahrweise kann der Katalysator (II) vorteilhaft im Reaktor verbleiben und das verbleibende Reaktionsgemisch dem Reaktor entnommen werden.

Die Abtrennung des Wasserstoffs kann in an sich bekannter Weise, vorzugsweise in einem Hochdruck-Abscheider, erfolgen. Dem Hochdruck-Abscheider kann vorteilhaft zum Abtrennen von gegebenenfalls verbleibendem Wasserstoff nachgeschaltet werden.

Vorteilhaft kann in Schritt b) Ammoniak ganz oder teilweise abgetrennt werden.

Die Abtrennung kann vorteilhaft destillativ erfolgen, insbesondere bei Sumpftemperaturen von 60 bis 220°C und Drücken von 1 bis 30 bar.

Nach Schritt b) wird eine Mischung (IV) erhalten.

Erfindungsgemäß setzt man gemäß Schritt c) eine Mischung (IV) gegebenenfalls in Gegenwart eines flüssigen Verdünnungsmittels (VII) in Gegenwart eines Katalysators (V) zu Caprolactam um.

Schritt c) kann vorteilhaft in Gegenwart eines flüssigen Verdünnungsmittels (VII) durchgeführt werden. Als Verdünnungsmittel (VII) kommen vorzugsweise Wasser oder organische Verdünnungsmittel beispielsweise C₁- bis C₉-Alkanole, wie Methanol, Ethanol, n-Propanol, Isopropanol, n-Butanol, i-Butanol, n-Pentanol, vorzugsweise aliphatische Kohlenwasserstoffe, wie n-Hexan, cycloaliphatische Kohlenwasserstoffe, wie Cyclopentan oder Cyclohexan, insbesondere aromatische Kohlenwasserstoffe, wie Benzol, Toluol, o-Xylol, m-Xylol, p-Xylol, Ethylbenzol, i-Propylbenzol, Di-i-propylbenzol sowie Gemische solcher Verbindungen, beispielsweise Petrolether, in Betracht. Die Kohlenwasserstoffe können funktionelle Gruppen, wie Halogene, beispielsweise Chlor, tragen, wie in Chlorbenzol.

Vorteilhaft können Verdünnungsmittel (VI) und Verdünnungsmittel (VII) gleich sein.

Als Katalysator (V) kommt vorteilhaft ein heterogener Katalysator in Betracht. Katalysator (V) kann dabei vorzugsweise als Festbettkatalysator eingesetzt werden.

Als Katalysatoren (V) sind saure, basische oder amphotere Oxide der Elemente der zweiten, dritten oder vierten Hauptgruppe des Periodensystems, wie Calciumoxid, Magnesiumoxid, Boroxid, Aluminiumoxid, Zinn-Oxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titandioxid, amorph, als Anatas oder Rutil, Zirkondioxid, Manganoxid oder Mischungen davon geeignet. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:

Vanadiumoxid, Bariumoxid, Zinkoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. dieses enthalten.

Weiterhin sind Zeolithe, Phosphate und Heteropolysäuren, sowie saure und alkalische Ionenaustauscher wie beispielsweise Nafion als geeignete Katalysatoren zu nennen.

Bevorzugte Katalysatoren sind Titanoxid, Aluminiumoxid, Ceroxid und Zirkondioxid, besonders bevorzugte Katalysatoren sind aktivierte Aluminiumoxide, wie sie beispielsweise aus WO 95/14664 bekannt sind.

Bei der Umsetzung gemäß Schritt c) kann auch Ammoniak anwesend, vorzugsweise abwesend sein.

Vorteilhaft beträgt in Schritt c) der Anteil der in Schritt a) aus Verbindung (I) entstandenen Verbindung (Verbindung (VIII)) an der Summe aus dieser Verbindung (VIII) und Verdünnungsmittel (VII) 0,1 bis 50 Gew.-%, vorzugsweise 1 bis 30 Gew.-%, insbesondere 2 bis 20 Gew.-%.

Die Umsetzung kann vorteilhaft in flüssiger Phase bei Temperaturen von im allgemeinen 140 bis 320°C, vorzugsweise 180 bis 300°C, besonders bevorzugt 200 bis 280°C durchgeführt werden. Der Druck sollte im allgemeinen im Bereich von 1 bis 250 bar, vorzugsweise von 5 bis 150 bar liegen.

Dabei sind solche Druck- und Temperaturbedingungen bevorzugt, unter denen das Reaktionsgemisch in einer einzigen homogenen flüssigen Phase vorliegt.

Die Katalysatorbelastungen liegen im allgemeinen im Bereich von 0,05 bis 5, vorzugsweise 0,1 bis 2, insbesondere 0,2 bis 1 kg Verbindung (VIII) / 1 Katalysatorvolumen / Stunde.

Bei der Umsetzung gemäß Schritt c) wird eine Mischung erhalten, die Caprolactam enthält. Diese Mischung enthält im allgemeinen neben Caprolactam Leichtsieder, Hochsieder, Verbindung (VIII), gegebenenfalls Ammoniak und Verdünnungsmittel (VII).

Unter Leichsieder werden dabei im Sinne der vorliegenden Erfindung Verbindungen mit einem Siedepunkt unterhalb dessen von Caprolactam verstanden, unter Hochsieder solche Verbindungen mit einem Siedepunkt oberhalb dessen von Caprolactam.

Aus dieser Mischung kann Caprolactam nach an sich bekannten Verfahren, wie durch Extraktion oder Destillation, erhalten werden. Im Falle der Destillation kann die Aufarbeitung vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Vorzugsweise kommt zunächst die Abtrennung von gegebenenfalls noch vorhandenem Ammoniak und Verdünnungsmittel (VII) von der Mischung in Betracht. Von der dann verbleibenden Mischung können dann die Hochsieder, Leichtsieder und gegebenenfalls nicht umgesetzte Verbindung (VIII) einzeln oder gemeinsam abgetrennt werden unter Erhalt von Caprolactam.

Vorteilhaft kann das bei dieser Aufarbeitung anfallende Verdünnungsmittel (VII) teilweise oder vollständig in Schritt a) oder Schritt c) zurückgeführt werden.

Vorteilhaft können bei dieser Aufarbeitung gegebenenfalls anfallende Hochsieder und/oder Leichtsieder teilweise oder vollständig in Schritt c) zurückgeführt werden.

Vorteilhaft kann bei dieser Aufarbeitung gegebenenfalls anfallende, nicht umgesetzte Verbindung (VIII) teilweise oder vollständig in Schritt c) zurückgeführt werden.

Das bei dem erfindungsgemäßen Verfahren erhaltene Caprolactam kann in an sich bekannter Weise bei der Herstellung von technisch wichtigen Polymeren, wie Polyamiden, eingesetzt werden.

### Beispiele

### a) Diskontinuierliche Hydrierung von Cyanvaleriansäuremethylester

In einem 0,27-Liter Autoklaven mit Drahtkorbeinsatz, indem sich jeweils 80 g Katalysatorformkörper befanden, und Begasungsrührer wurden gemäß Tabelle 1 Cyanvaleriansäuremethylester, Lösungsmittel und Ammoniak als flüssige Phase vorgelegt unter den dort genannten Reaktionsbedingungen umgesetzt. Der entsprechende Reaktionsdruck wurde durch kontinuierliche Wasserstoffzudosierung konstant gehalten.

Die Ergebnisse sind Tabelle 1 zu entnehmen.

**Tabelle 1**

| Kat. | Edukte [g] | | | Reaktionsbeding. | | | Produkte Ausbeute in [%] | | |
|---|---|---|---|---|---|---|---|---|---|
| | CVSE | LM | NH₃ | T | P | t | ACSME | ACSA | CL |
| | | (79g) | | [°C] | [bar] | [min] | | | |
| Magnetit | 8,8 | MTBE | 8,1 | 110 | 250 | 30 | 92,4 | 3,0 | 3,6 |
| Magnetit | 17 | Toluol | 8,1 | 110 | 250 | 35 | 96,7 | 1,8 | 1,6 |
| Raney-Ni | 17 | -- | 69 | 50 | 100 | 220 | 91,0 | 5,9 | 0,3 |
| Cobalt | 17 | -- | 69 | 90 | 250 | 30 | 93,8 | 3,7 | 0,9 |
| Cobalt | 17 | -- | 69 | 50 | 100 | 70 | 98,2 | 0,8 | 0,3 |
| Cobalt | 8,8 | Toluol | 69 | 50 | 100 | 165 | 93,9 | -- | 1,1 |
| Cobalt | 8,8 | Toluol | 8,1 | 50 | 100 | 160 | 94,0 | 0,6 | 1,2 |
| Raney-Co | 17 | -- | 69 | 50 | 100 | 45 | 91,7 | 1,1 | 1,1 |
| Raney-Co | 8,8 | Toluol | -- | 50 | 100 . | 65 | 93,6 | -- | 0,3 |
| Hydrotalcit | 8,8 | Toluol | 8,1 | 50 | 100 | 22 | 90,5 | 3,9 | 3,4 |
| Raney-Co | 8,8 | Toluol | 8,8 | 60 | 100 | 90 | 98,1 | 1,0 | 0,6 |
| Raney-Co | 8,8 | Toluol | 19,6 | 60 | 100 | 90 | 98,8 | 0,8 | 0,3 |

| | | | | | | | | | |
|---|---|---|---|---|---|---|---|---|---|
| CVSE: 6-Cyanvaleriansäuremethylester ACSME: 6-Aminocapronsäuremethylester ACSA: 6-Aminocapronsäureamid CL: Caprolactam MTBE: Methyl-t-butyl-ether | | | | | | | | | |

### b) Diskontinuierliche Cyclisierung von 6-Aminocapronsäuremethylester zu Caprolactam

2 ml (1,6 g) einer 10 Gew.-% Lösung von 6-Aminocapronsäuremethylester in Toluol wurde gemäß Tabelle 2 ohne bzw. mit jeweils 1 g Katalysatorpulver in einem 5 ml Autoklaven bei 150°C umgesetzt.

Die Umsätze von 6-Aminocapronsäuremethylester zu Caprolactam in [%] sind Tabelle 2 zu entnehmen.

**Tabelle 2**

| | Katalysator | | | | | | |
|---|---|---|---|---|---|---|---|
| t [min] | Keiner | SiO₂ | g-Al₂O₃ | TiO₂ | ZrO₂ | MgO | CaO |
| 15 | 4,4 | 46,9 | 70,3 | 53,1 | 43,7 | 8,1 | 4,7 |
| 30 | 5,2 | 69,7 | 89,1 | 73,6 | 63,1 | 11,6 | 5,8 |
| 45 | 5,9 | 80,0 | 92,4 | 81,2 | 71,5 | 14,5 | 6,2 |
| 60 | 7,0 | 83,1 | 94,2 | 84,5 | 76,7 | 17,7 | 7,7 |
| 120 | 11,6 | 98,7 | 99,0 | 98,0 | 98,0 | 28,6 | 13,0 |

### c) Kontinuierliche Hydrierung von Cyanvaleriansäuremethylester

### 1. An einem Cobalt-Katalysator

In einem 100 ml-Rohrreaktor mit separatem Zulauf für Eduktlösung, Ammoniak und Wasserstoff wurden 100ml Cobalt-Katalysator bei 280°C aktiviert und bei einem Druck von 200 bar und einem Ammoniak/CVSE-Verhältnis von 1:1 die Belastung (in g CVSE / ml Katalysator / Stunde) und Temperatur gemäß Tabelle 3 variiert.

Die Ergebnisse sind Tabelle 3 zu entnehmen.

**Tabelle 3**

| Belastung | T [°C] | CVSE-Umsatz [%] | ACSME-Selektivität [%] |
|---|---|---|---|
| 0,3 | 80 | 94,60 | 94,46 |
| 0,15 | 80 | 98,64 | 98,63 |
| 0,2 | 80 | 95,31 | 95,31 |
| 0,2 | 90 | 98,46 | 98,08 |
| 0,2 | 100 | 99,36 | 99,04 |
| 0,2 | 120 | 99,92 | 99,32 |

| | | | |
|---|---|---|---|
| CVSE: 6-Cyanvaleriansäuremethylester ACSME: 6-Aminocapronsäuremethylester | | | |

### 2. An einem mit Cr dotierten Raney-Cobalt

In einem 100 ml-Rohrreaktor mit separatem Zulauf für Eduktlösung, Ammoniak und Wasserstoff und 100 ml Cr-dotiertem Raney-Co wurde bei einem Druck von 80 bar, einer Temperatur von 80°C und einem Ammoniak/CVSE-Verhältnis von 2:1 die Belastung (in g CVSE / ml Katalysator / Stunde) gemäß Tabelle 4 variiert.

Die Ergebnisse sind Tabelle 4 zu entnehmen.

**Tabelle 4**

| Belastung | CVSE-Umsatz [%] | ACSME-Selektivität [%] |
|---|---|---|
| 0,5 | 64,80 | 64,77 |
| 0,4 | 80,30 | 80,30 |
| 0,3 | 89,50 | 89,50 |
| 0,25 | 98,50 | 98,61 |
| 0,2 | 99,60 | 99,56 |

| | | |
|---|---|---|
| CVSE: 6-Cyanvaleriansäuremethylester ACSME: 6-Aminocapronsäuremethylester | | |

### d) Kontinuierliche Cyclisierung von 6-Aminocapronsäuremethylester zu Caprolactam

### 1. An einem TiO₂-Katalysator

Eine 10 Gew.-% Lösung von 6-Aminocapronsäuremethylester in Toluol, die gemäß Tabelle 5 Wasser enthalten konnte, wurde an einem TiO₂-Katalysator (1,5 mm Stränge) in einem 20 ml-Röhrenreaktor (90 cm lang, 6 mm Durchmesser) bei Reaktionstemperatur und Belastung (in kg / 1 / h) gemäß Tabelle 5 umgesetzt.

Die Ergebnisse sind Tabelle 5 zu entnehmen.

**Tabelle 5**

| Belastung | T | Wasser | ACSME-Umsatz [%] | CL-Selektivität |
|---|---|---|---|---|
| | [°C] | [Gew-%] | | [%] |
| 0,5 | 200 | 0 | 95,44 | 94,54 |
| 0,4 | 200 | 0 | 97,73 | 95,24 |
| 0,5 | 220 | 0 | 98,30 | 96,85 |
| 0,5 | 220 | 0 | 98,64 | 96,23 |
| 0,5 | 220 | 5 | 98,93 | 96,44 |
| 0,5 | 250 | 0 | 97,56 | 94,95 |
| 0,5 | 240 | 0 | 96,87 | 95,85 |
| 0,5 | 230 | 0 | 97,42 | 97,31 |
| 0,5 | 220 | 0 | 97,92 | 96,58 |

| | | | | |
|---|---|---|---|---|
| ACSME: 6-Aminocapronsäuremethylester CL: Caprolactam | | | | |

### 2. An einem gamma-Aluminiumoxid-Katalystor

Eine 10 Gew.-% Lösung von 6-Aminocapronsäuremethylester in Toluol, die gemäß Tabelle 5 Wasser enthalten konnte, wurde an einem gamma-Aluminiumoxid-Katalystor (1-3 mm Splitt) in einem 20 ml-Röhrenreaktor (90 cm lang, 6 mm Durchmesser) bei Reaktionstemperatur und Belastung (in kg / 1 / h) gemäß Tabelle 6 umgesetzt.

Die Ergebnisse sind Tabelle 6 zu entnehmen.

**Tabelle 6**

| Belastung | T | Wasser | ACSME-Umsatz [%] | CL-Selektivität |
|---|---|---|---|---|
| | [°C] | [Gew-%] | | [%] |
| 0,5 | 220 | 0 | 97,86 | 96,36 |
| 0,55 | 220 | 0 | 97,87 | 95,84 |
| C,6 | 220 | 0 | 98,04 | 96,49 |

| | | | | |
|---|---|---|---|---|
| ACSME: 6-Aminocapronsäuremethylester CL: Caprolactam | | | | |

## Patentansprüche

1. Verfahren zur Herstellung von Caprolactam aus einer Verbindung der Formel (I)
NC - (CH₂)₄ - CO - R (I)
mit R: Carbonsäureamid-, Carbonsäure- oder Carbonsäureestergruppe
**dadurch gekennzeichnet, daß** man
a) eine Verbindung (I) oder deren Gemische in Gegenwart von Ammoniak und gegebenenfalls eines flüssigen Verdünnungsmittels (VI) mit Wasserstoff in Gegenwart eines Katalysators (II), wobei als katalytisch aktive Komponente des Katalysators (II) ein Metall, ausgewählt aus der Gruppe bestehend aus Fe, Ni, Co oder Ru oder deren Gemische, verwendet wird, hydriert unter Erhalt einer Mischung (III),
b) aus Mischung (III) Wasserstoff und Katalysator (II) abtrennt unter Erhalt einer Mischung (IV) und
c) Mischung (IV) gegebenenfalls in Gegenwart eines flüssigen Verdünnungsmittels (VII) in Gegenwart eines Katalysators (V) zu Caprolactam umsetzt.

2. Verfahren nach Anspruch 1, wobei Katalysator (II) ein heterogener Katalysator ist.

3. Verfahren nach Anspruch 1 oder 2, wobei Katalysator (II) ein Festbett-Katalysator ist.

4. Verfahren nach den Ansprüchen 1-3, wobei das in Stufe a) eingesetzte Reaktionsgemisch bei der Hydrierung flüssig vorliegt.

5. Verfahren nach den Ansprüchen 1-4, wobei in Schritt b) zusätzlich Ammoniak ganz oder teilweise abgetrennt wird.

6. Verfahren nach den Ansprüchen 1-5, wobei Katalysator (V) als katalytisch aktive Komponente ein Metalloxid enthält.

7. Verfahren nach den Ansprüchen 1-6, wobei Katalysator (V) ein Festbett-Katalysator ist.

8. Verfahren nach den Ansprüchen 1-7, wobei als katalytisch aktive Komponente des Katalysators (V) ein Metalloxid ausgewählt aus der Gruppe bestehend aus Titandioxid, Aluminiumoxid, Ceroxid und Zirkonoxid oder deren Gemische, eingesetzt wird.

## Claims

1. A process for the preparation of caprolactam from a compound of formula (I):
NC - (CH₂)₄ - CO - R (I)
in which R is a carboxamide, carboxylic acid or carboxylic acid ester group,
wherein
a) a compound (I) or a mixture of such compounds, in the presence of ammonia and if appropriate a liquid diluent (VI), is hydrogenated with hydrogen in the presence of a catalyst (II), wherein a metal selected from the group comprising Fe, Ni, Co and Ru, or mixtures thereof, is used as the catalytically active component of the catalyst (II), to give a mixture (III),
b) the hydrogen and the catalyst (II) are separated from the mixture (III) to give a mixture (IV), and
c) the mixture (IV), if appropriate in the presence of a liquid diluent (VII), is converted to caprolactam in the presence of a catalyst (V).

2. The process according to claim 1 wherein the catalyst (II) is a heterogeneous catalyst.

3. The process according to claim 1 or 2 wherein the catalyst (II) is a fixed bed catalyst.

4. The process according to any of claims 1 to 3 wherein the reaction mixture used in step a) is in liquid form during the hydrogenation.

5. The process according to any of claims 1 to 4 wherein all or part of the ammonia is additionally separated off in step b).

6. The process according to any of claims 1 to 5 wherein the catalyst (V) comprises a metal oxide as the catalytically active component.

7. The process according to any of claims 1 to 6 wherein the catalyst (V) is a fixed bed catalyst.

8. The process according to any of claims 1 to 7 wherein a metal oxide selected from the group comprising titanium dioxide, aluminum oxide, cerium oxide and zirconium oxide, or mixtures thereof, is used as the catalytically active component of the catalyst (V).

## Revendications

1. Procédé de préparation de caprolactame à partir d'un composé de la formule (I) :
NC - (CH₂)₄ - CO - R (I)
où R est un groupe amide d'acide carboxylique, acide carboxylique ou ester d'acide carboxylique,
**caractérisé en ce que**
a) on hydrogène un composé (I) ou des mélanges de ces derniers en présence d'ammoniac et éventuellement d'un diluant liquide (VI) avec de l'hydrogène en présence d'un catalyseur (II), en utilisant, comme composant catalytiquement actif du catalyseur (II), un métal choisi parmi le groupe constitué de Fe, Ni, Co ou Ru ou leurs mélanges, ce qui donne un mélange (III),
b) on isole du mélange (III) de l'hydrogène et du catalyseur (II), avec obtention d'un mélange (IV), et
c) on convertit du mélange (IV) en caprolactame, éventuellement en présence d'un diluant liquide (VII) en présence d'un catalyseur (V).

2. Procédé suivant la revendication 1, dans lequel le catalyseur (II) est un catalyseur hétérogène.

3. Procédé suivant la revendication 1 ou 2, dans lequel le catalyseur (II) est un catalyseur à lit fixe.

4. Procédé suivant les revendications 1 à 3, dans lequel le mélange réactionnel mis en oeuvre dans l'étape a) se présente à l'état liquide lors de l'hydrogénation.

5. Procédé suivant les revendications 1 à 4, dans lequel, dans l'étape b), on isole en supplément, totalement ou partiellement, de l'ammoniac.

6. Procédé suivant les revendications 1 à 5, dans lequel le catalyseur (V) contient un oxyde métallique comme composant catalytiquement actif.

7. Procédé suivant les revendications 1 à 6, dans lequel le catalyseur (V) est un catalyseur à lit fixe.

8. Procédé suivant les revendications 1 à 7, dans lequel, comme composant catalytiquement actif du catalyseur (V), on met en oeuvre un oxyde métallique choisi parmi le groupe constitué du dioxyde de titane, de l'oxyde d'aluminium, de l'oxyde de cérium et de l'oxyde de zirconium ou de leurs mélanges.
